# EUROPEAN PATENT APPLICATION

(11) **EP 4 799 668 A1**
(43) Date of publication of application: **02.09.2026**
(21) Application number: 24896423.1
(22) Date of filing: 22.11.2024
(51) Int. Cl.: A61M 11/00

(54) **MICRO-MESH ATOMIZATION UNIT ASSEMBLY, ATOMIZER SYSTEM, AND CONTROL METHOD**

(30) Priority: 30.11.2023 CN 202311625524
(71) Applicant: Qingdao Future Medical Technology Co., Ltd., Qingdao, Shandong 266102 (CN)
(72) Inventor: WANG, Weilai, Qingdao, Shandong 266102 (CN); WANG, Qixu, Qingdao, Shandong 266102 (CN); SUN, Zhongjin, Qingdao, Shandong 266102 (CN)
(74) Representative: Zacco GmbH
(86) International application number: PCT/CN2024/133730
(87) International publication number: WO 2025/113332

(57) **Abstract**

A mesh nebulization unit assembly, a nebulizer system and a control method. The mesh nebulization unit assembly includes a mesh nebulization sheet (1) and a housing with a hole formed in a middle, wherein a periphery of the mesh nebulization sheet (1) is packaged in the housing, at least three groups of nebulization sheet positioning ribs are disposed inside the housing to clamp an edge of the mesh nebulization sheet (1), and a certain gap is maintained between a non-clamped portion on the mesh nebulization sheet (1) and the housing, and a size of a clamping opening formed by each group of nebulization sheet positioning ribs is equal to or less than a thickness of the mesh nebulization sheet (1). The nebulizer system includes a mesh nebulization unit assembly, a liquid medicine cup, a mist nozzle and a nebulizer controller. By means of appropriate structural settings of the mesh nebulization unit assembly, the problems of reduced nebulization ability and energy loss caused by the encapsulation of the nebulization sheet can be avoided, and the service life is relatively long. The control method of a nebulizer may ensure that a nebulization work can be performed only when the nebulization unit assembly and the nebulizer controller are mutually authenticated and meet use conditions, thereby preventing abuse of the mesh nebulization sheet.

## Description

### Technical Field

The present disclosure belongs to the technical field of medical devices, and relates to improvements to a medical nebulizer and a control method, in particular to a mesh nebulization unit assembly, a nebulizer system and a control method.

### Background

A nebulizer is an important device for treating respiratory diseases and is widely used in vaccination and the treatment of various non-respiratory diseases, thereby having broad market prospects. At present, a mesh nebulizer is the mainstream, and the mesh nebulizer is composed of a liquid medicine cup, a nebulization sheet, a mouthpiece and a controller, and the nebulization sheet is a core component. The nebulization sheet is formed by attaching piezoelectric ceramic to a microporous mesh sheet, mechanical energy is generated by the piezoelectric ceramic under an excitation of an electrical signal source, so as to drive the microporous mesh sheet to vibrate, so that liquid medicine is quickly screened, vibrated and ejected from tiny mesh holes on the mesh sheet to uniformly form numerous tiny nebulization particles to achieve the purpose of nebulization.

In order to ensure the nebulization therapy effect, it is necessary to disassemble, maintain, clean, disinfect and even replace the nebulization sheet in the use of the nebulizer. Thus, a nebulizer design is to encapsulate the nebulization sheet as an independent unit assembly to facilitate disassembly and assembly, cleaning, disinfection, maintenance and replacement of the nebulization sheet as necessary.

However, existing mesh nebulization unit assemblies generally have the problems of reduced nebulization ability and energy loss caused by encapsulation, and the energy loss may also result in a temperature rise of the nebulization sheet to affect the nebulization effect, and also lead to fatigue failure and even breakage of the nebulization sheet and a connecting piece thereof.

Therefore, technical problems to be solved urgently in the art involve designing a mesh nebulization unit assembly and a nebulizer system that are capable of avoiding reduced nebulization ability and energy loss of a mesh nebulization sheet caused by the encapsulation of the mesh nebulization sheet, and ensuring a relatively long service life of the mesh nebulization sheet; and in addition, enabling the nebulizer system to realize wireless power supply to the mesh nebulization sheet and perform mutual authentication with the nebulization unit assembly to realize intelligent control.

### Summary

In order to solve the above problems in the prior art, an embodiment of the present disclosure provides a mesh nebulization unit assembly, a nebulizer system and a control method. The mesh nebulization unit assembly has a reasonable structure, may avoid the problems of reduced nebulization ability and energy loss caused by the encapsulation of a nebulization sheet, and has a relatively long service life; and the nebulizer control method may ensure that a nebulization work can be performed only when a nebulization unit assembly and a nebulizer controller are mutually authenticated and meet use conditions, thereby preventing abuse of the mesh nebulization sheet.

The objective of the present disclosure is implemented by the following technical solution:
A mesh nebulization unit assembly, including a mesh nebulization sheet, and mesh holes being formed in a middle of the mesh nebulization sheet, wherein the mesh nebulization unit assembly further includes a housing with an open pore formed in a middle, a periphery of the mesh nebulization sheet is encapsulated in the housing, the mesh hole in the middle of the mesh nebulization sheet is exposed from the open pore in the middle of the housing, at least three groups of nebulization sheet positioning ribs are disposed inside the housing to clamp an edge of the mesh nebulization sheet, a certain gap is maintained between a non-clamped portion of the mesh nebulization sheet and the housing, and a size of a clamping opening formed by each group of nebulization sheet positioning ribs is equal to or less than a thickness of the mesh nebulization sheet.

Another improvement to the above technical solution: the housing is made of a hard material, a nebulization sheet clamping surface of the each group of nebulization sheet positioning ribs is made of elastic soft rubber, and the size of the clamping opening formed by the each group of nebulization sheet positioning ribs is slightly less than the thickness of the mesh nebulization sheet.

Further improvements to the above technical solution: the mesh nebulization sheet is composed of a steel sheet provided with the mesh hole at a center, an FPC and a piezoelectric ceramic, a ribbon-shaped strip of an integrated structure extends out from an edge of the steel sheet, a steel sheet welding point for connecting with a PIN is disposed at a tail end of the ribbon-shaped strip, and the ribbon-shaped strip is provided with a bent structure between the steel sheet welding point and the edge of the steel sheet; and a strip-shaped ribbon of an integrated structure also extends out from an edge of the FPC, an FPC welding point for connecting with the PIN is disposed at a tail end of the strip-shaped ribbon, and the strip-shaped ribbon is provided with a bent structure between the FPC welding point and the edge of the FPC.

Further improvements to the above technical solution: the bent structure of the ribbon-shaped strip between the steel sheet welding point and the edge of the steel sheet is a U-shaped bend, and a length of the U-shaped bend on the ribbon-shaped strip is set to be a non-integer multiple of 1/4 of a vibration wavelength of the mesh nebulization sheet; and the bent structure of the strip-shaped ribbon between the FPC welding point and the edge of the FPC is a U-shaped bend, and a length of the U-shaped bend on the strip-shaped ribbon is set to be a non-integer multiple of 1/4 of the vibration wavelength of the mesh nebulization sheet.

Further improvements to the above technical solution: the steel sheet is circular, the FPC and the piezoelectric ceramic are both circular ring-shaped, a size of the FPC is greater than a size of the piezoelectric ceramic, the FPC completely covers the piezoelectric ceramic, and both sides of the FPC are adhered to the steel sheet, so that the piezoelectric ceramic is completely enclosed between the FPC and the steel sheet.

Further improvements to the above technical solution: the housing is circular ring-shaped and includes a circular ring-shaped upper shell and a circular ring-shaped lower shell, the mesh nebulization sheet is assembled at an annular center of the housing, at least three nebulization sheet positioning ribs I protruding from an inner edge of the housing are uniformly disposed on an annular inner edge of the upper shell, the at least three nebulization sheet positioning ribs have consistent outlines and are all circular arc-shaped, the lower shell is provided with nebulization sheet positioning ribs II having completely consistent outlines and opposite clamping directions at locations corresponding to the nebulization sheet positioning ribs of the upper shell, an annular outer edge of the lower shell is a certain thickness higher than an annular inner edge thereof, and after the lower shell is assembled with the upper shell, the lower shell completely wraps the outer edge of the upper shell.

Further improvements to the above technical solution: an edge of the upper shell is provided with at least one upper shell positioning groove, an inner side of the annular outer edge of the lower shell is correspondingly provided with a lower shell positioning protrusion, and the shape and size of the lower shell positioning protrusion completely match those of the upper shell positioning groove.

Further improvements to the above technical solution: the annular inner edge of the lower shell is also provided with a certain height, so that an annular space between the annular outer edge of the lower shell and the annular inner edge thereof forms a glue dispensing groove for injecting glue to bond various portions of the mesh nebulization unit assembly into a whole.

Further improvements to the above technical solution: an inner bottom surface of the glue dispensing groove is provided with a PIN mounting hole, the PIN mounting hole is a hollow cylinder extending downwards starting from the inner bottom surface of the glue dispensing groove, a hollow inner diameter of the hollow cylinder matches an outer diameter of the inserted PIN, and the PIN is provided with a pin cap having a diameter greater than that of a pin foot.

Further improvements to the above technical solution: the mesh nebulization unit assembly is provided with an electric energy wireless receiving apparatus, the electric energy wireless receiving apparatus is completely encapsulated in the housing, the electric energy wireless receiving apparatus is connected with the mesh nebulization sheet, and is used for receiving electric energy emitted by an external electric energy wireless sending apparatus, so as to realize wireless power supply to the mesh nebulization sheet.

A wireless power supply nebulizer system, including a nebulization unit assembly, a liquid medicine cup, a mist nozzle and a nebulizer controller, wherein the nebulization unit assembly is the above mesh nebulization unit assembly, the nebulizer controller is provided with an electric energy wireless sending apparatus, the electric energy wireless sending apparatus is used for providing electric energy for the electric energy wireless receiving apparatus in the mesh nebulization unit assembly by means of a control of the nebulizer controller, and the electric energy wireless receiving apparatus drives the mesh nebulization sheet in the mesh nebulization unit assembly to work.

An intelligent control nebulizer system, including a nebulization unit assembly, a liquid medicine cup, a mouthpiece and a nebulizer controller, wherein the nebulization unit assembly is the above mesh nebulization unit assembly, the mesh nebulization unit assembly further includes a control apparatus and an electric energy apparatus, and the control apparatus includes a communication module, a single chip microcomputer and a control module running thereon, the control module and the communication module are configured to perform wired or wireless communication with the nebulizer controller to jointly control the mesh nebulization sheet in the mesh nebulization unit assembly to work; and the electric energy apparatus uses a PIN wired connection power supply mode, a wireless power supply mode of an electric energy wireless receiving apparatus, or a power supply mode of a rechargeable battery to realize partial or complete power supply to the control apparatus and the mesh nebulization sheet.

Further improvements to the above technical solution: the communication module in the control apparatus is a wireless communication module, the nebulizer controller is provided with the wireless communication module and an electric energy wireless sending apparatus, the electric energy wireless receiving apparatus and the control apparatus are completely encapsulated in the housing in the mesh nebulization unit assembly, the electric energy wireless sending apparatus is used for providing electric energy for the electric energy wireless receiving apparatus in the mesh nebulization unit assembly by means of the control of the nebulizer controller, and the electric energy wireless receiving apparatus drives the mesh nebulization sheet in the mesh nebulization unit assembly to work.

The present disclosure provides a control method of the intelligent control nebulizer system, wherein the control method includes the following steps:
step 1: after the nebulizer system is powered on and started, the mesh nebulization unit assembly exchanging identity authentication information with the nebulizer controller by means of the communication module, so as to confirm whether the other party meets identity requirements of a connection work, and only when the nebulizer controller meets a preset allowed-to-use identity condition, the mesh nebulization unit assembly can complete an identity authentication operation and enter the next step; and otherwise, sending a refuse-to-work warning to the nebulizer controller, and prompting a cause in a warning message;
step 2: checking a current state of the mesh nebulization sheet, and according to a preset starting condition of the mesh nebulization sheet, determining whether the mesh nebulization sheet is in an allowed-to-start state, wherein the starting condition of the mesh nebulization sheet includes whether a service life of the mesh nebulization sheet has expired, and whether there is an abnormal record in an operation log that affects a working of the mesh nebulization sheet; when the check is passed and the starting condition of the mesh nebulization sheet is met, notifying the nebulizer controller that the mesh nebulization unit assembly is ready, and entering a next step to wait for a control command from the nebulizer controller; and otherwise, sending a refuse-to-work warning to the nebulizer controller, and prompting a cause in the warning message;
step 3: waiting for the control command of the nebulizer controller, and after receiving a command from the nebulizer controller to control the mesh nebulization sheet to start, starting the mesh nebulization sheet to start working;
step 4: during a working of the mesh nebulization sheet, the control apparatus checking, in real time, whether working parameters of a driving circuit of the mesh nebulization sheet are optimal, wherein the working parameters include driving power and working frequency; and adjusting input parameters of the driving circuit of the mesh nebulization sheet according to a preset condition, so as to ensure that the mesh nebulization sheet works in an optimal state; and
step 5: the control apparatus monitoring a working state of the mesh nebulization sheet, and recording working conditions and log information of the mesh nebulization sheet in real time, including the number of times of use, time information of each use, operation information of the mesh nebulization sheet, and the information of a corresponding nebulizer controller.

Compared with the prior art, an embodiment of the present disclosure has the following advantages and positive effects:
1. the pressure clamping of the mesh nebulization sheet in the mesh nebulization unit assembly in the embodiment of the present disclosure is very small, and the rest of the mesh nebulization sheet is not in contact with the housing, thereby maximally reducing the influence of the housing on the working of the nebulization sheet, and avoiding the problems of reduced nebulization ability and energy loss caused by the encapsulation of the nebulization sheet;
2. appropriate bends are introduced to a welding point of the nebulization sheet and the edge of the nebulization sheet in the embodiment of the present disclosure, thereby avoiding anomalies such as desoldering and breakage of the steel sheet due to the fact that the vibration of the nebulization sheet during working is conducted to the welding point, further reducing unnecessary energy loss, and optimizing the nebulization ability of the nebulization unit;
3. the piezoelectric ceramic of the mesh nebulization sheet in the embodiment of the present disclosure is completely encapsulated, thereby avoiding the possibility of precipitation of substances contained in the piezoelectric ceramic in a nebulization process, and improving the safety of the mesh nebulization sheet;
4. the mesh nebulization unit assembly in the present disclosure has a simple and reasonable structure, is simple and convenient to assemble, is not prone to error, and has a high yield;
5. according to the mesh nebulization unit assembly capable of realizing wireless power supply in the embodiment of the present disclosure, the electric energy wireless receiving apparatus is completely encapsulated in the housing, thereby reducing unnecessary exposure, improving the waterproof and dustproof performance of the mesh nebulization unit assembly, and prolonging the service life of the mesh nebulization unit assembly;
6. the intelligent mesh nebulization unit assembly and the nebulizer system in the embodiment of the present disclosure have the advantages of preventing the abuse of the nebulization sheet, ensuring special equipment for special use, monitoring in real time and dynamically adjusting control parameters of the nebulization sheet to ensure the optimal working state of the nebulization sheet; and
7. the control method of the intelligent nebulizer system in the present disclosure may ensure that a nebulization work can be performed only when the nebulization unit assembly and the nebulizer controller are mutually authenticated and meet use conditions, thereby preventing abuse of the mesh nebulization sheet.

### Brief Description of the Drawings

Fig. 1 is a perspective view for illustrating one side of an upper shell of a mesh nebulization unit assembly in the present disclosure;
Fig. 2 is a perspective view for illustrating one side of a lower shell of a mesh nebulization unit assembly in the present disclosure;
Fig. 3 is a top view for illustrating one side of a lower shell of a mesh nebulization unit assembly in the present disclosure;
Fig. 4 is a sectional view in a direction A-A of Fig. 3;
Fig. 5 is a perspective view of a mesh nebulization sheet in a mesh nebulization unit assembly in the present disclosure;
Fig. 6 is a top view of a mesh nebulization sheet in a mesh nebulization unit assembly in the present disclosure;
Fig. 7 is a sectional view in a direction B-B of Fig. 6;
Fig. 8 is an exploded view of assembly of a mesh nebulization sheet in a mesh nebulization unit assembly in the present disclosure;
Fig. 9 is an exploded view of assembly of a mesh nebulization unit assembly in the present disclosure;
Fig. 10 is a schematic structural diagram of a wireless power supply nebulizer system in the present disclosure;
Fig. 11 is a schematic structural diagram of an intelligent mesh nebulization unit assembly in the present disclosure;
Fig. 12 is a schematic structural diagram of a nebulizer system for mounting an intelligent mesh nebulization unit assembly in the present disclosure;
Fig. 13 is a block diagram of a control flow of an intelligent mesh nebulization unit assembly in the present disclosure;
Fig. 14 is a block diagram of a verification flow of verification and use restriction conditions of an intelligent mesh nebulization unit assembly and a nebulizer controller in the present disclosure; and
Fig. 15 is a block diagram of a control flow of an intelligent mesh nebulization unit assembly receiving an operation request from a nebulizer controller in the present disclosure.

Reference signs in the figures: 1, mesh nebulization sheet; 1.1, glue; 1.2, steel sheet; 1.3, FPC; 1.4, piezoelectric ceramic; 1.5, mesh hole; 1.6, steel sheet welding point; 1.7, FPC welding point; 2, upper shell; 2.1, nebulization sheet positioning rib I; 2.2, upper shell positioning groove; 3, lower shell; 3.1, nebulization sheet positioning rib II; 3.2, lower shell positioning protrusion; 3.3, PIN mounting hole; 3.4, glue dispensing groove; 4, PIN.

### Detailed Description of the Embodiments

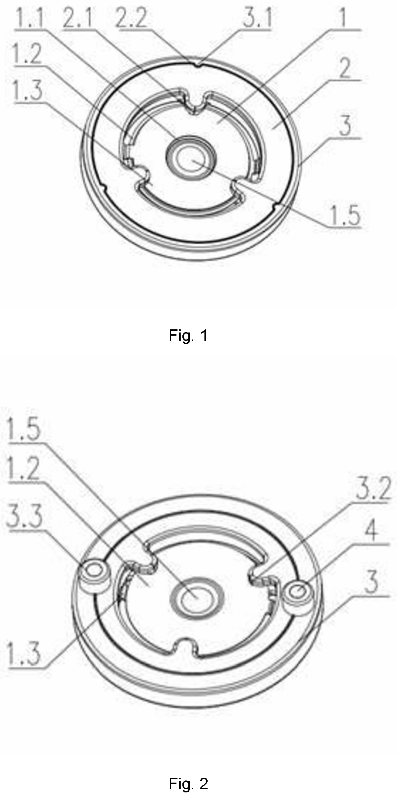

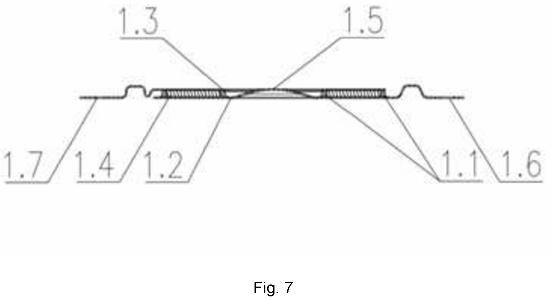

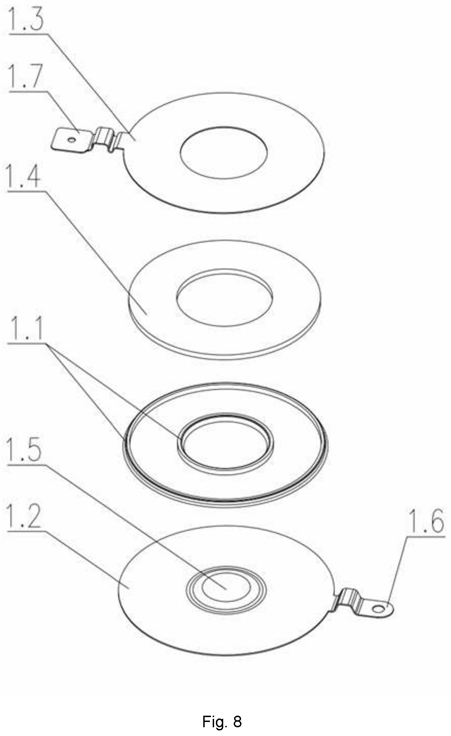

The present disclosure is further described in detail below in combination with the drawings:
Referring to Fig. 1 to Fig. 9, an embodiment of a mesh nebulization unit assembly in the present disclosure includes a mesh nebulization sheet 1 and a housing with an open pore formed in a middle, mesh holes 1.5 are formed in a middle of the mesh nebulization sheet 1, a periphery of the mesh nebulization sheet 1 is encapsulated in the housing, and the mesh holes 1.5 in the middle of the mesh nebulization sheet 1 are exposed from the open pore in the middle of the housing. At least three groups of nebulization sheet positioning ribs are disposed inside the housing to clamp an edge of the mesh nebulization sheet 1, a certain gap is maintained between a non-clamped portion of the mesh nebulization sheet 1 and the housing, two nebulization sheet positioning ribs disposed opposite to each other up and down form one group, and a size of a clamping opening formed by each group of nebulization sheet positioning ribs is equal to or less than a thickness of the mesh nebulization sheet 1.

Specifically, the housing is made of a hard material, a nebulization sheet clamping surface of the nebulization sheet positioning rib is made of elastic soft rubber, and the size of the clamping opening formed by the each group of nebulization sheet positioning ribs is slightly less than the thickness of the mesh nebulization sheet 1, so that mesh nebulization sheet 1 bears a very small clamping force. Thus, not only can the stability of clamping the mesh nebulization sheet be increased, but also restrictions on the housing on the working of the mesh nebulization sheet 1 are avoided, thereby avoiding the problems of reduced nebulization ability and energy loss caused by the encapsulation of the mesh nebulization sheet 1.

In some embodiments, the mesh nebulization sheet 1 is composed of a steel sheet 1.2 provided with the mesh holes 1.5 at the center, a flexible printed circuit board (FPC) 1.3 and piezoelectric ceramic 1.4, a ribbon-shaped strip of an integrated structure extends out from an edge of the steel sheet 1.2, a steel sheet welding point 1.6 for connecting with a PIN 1.4 is disposed at a tail end of the ribbon-shaped strip, and the ribbon-shaped strip is provided with a bent structure between the steel sheet welding point 1.6 and the edge of the steel sheet 1.2. In this way, situations such as desoldering and breakage due to the fact that the vibration of the mesh nebulization sheet 1 during working is conducted to the steel sheet welding point 1.6 can be avoided, thereby reducing related energy loss, and further optimizing the nebulization ability of the mesh nebulization sheet 1. A strip-shaped ribbon of an integrated structure also extends out from an edge of the FPC 1.3, an FPC welding point 1.7 for connecting with the PIN 4 is disposed at a tail end of the strip-shaped ribbon, and the strip-shaped ribbon is provided with a bent structure between the FPC welding point 1.7 and the edge of the FPC 1.3. In this way, anomalies such as desoldering due to the fact that the vibration of the mesh nebulization sheet 1 during working is conducted to the FPC welding point 1.7 can be avoided, meanwhile, related unnecessary energy loss is reduced, and the nebulization ability is further optimized.

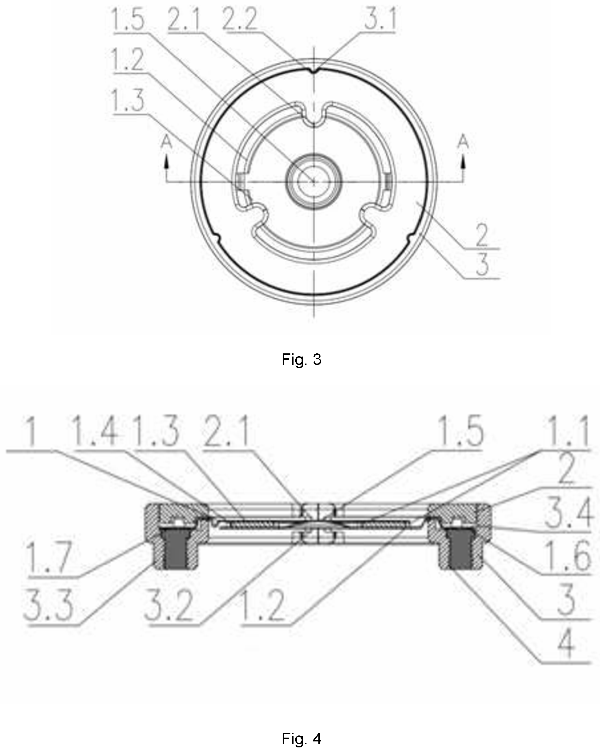

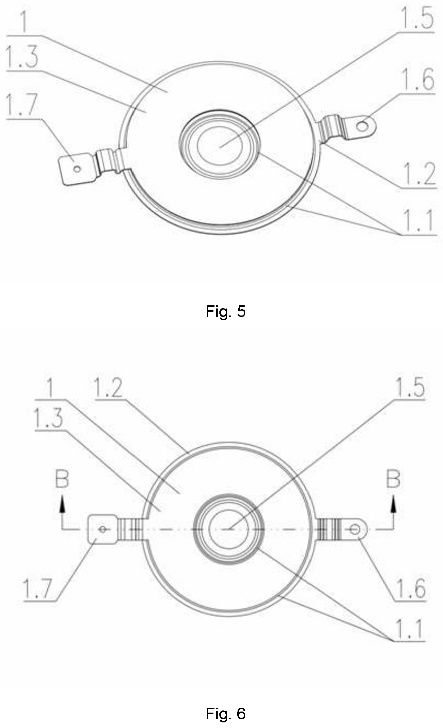

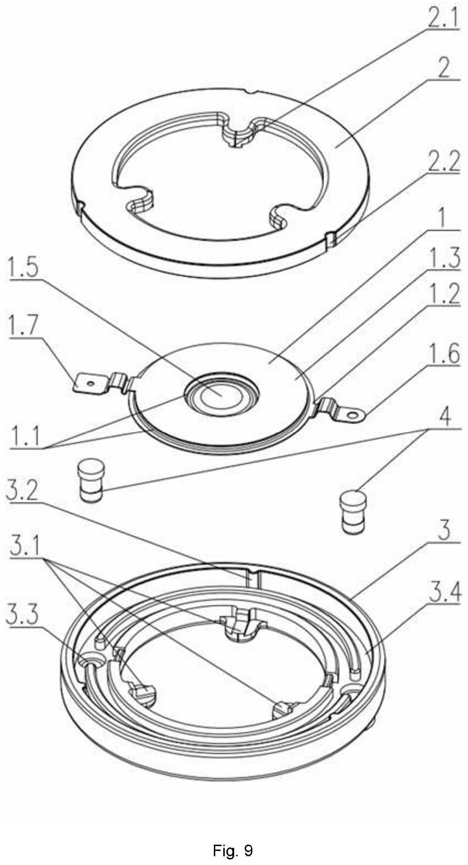

Further, as shown in Fig. 5, Fig. 7 and Fig. 8, the bent structure of the ribbon-shaped strip between the steel sheet welding point 1.6 and the edge of the steel sheet 1.2 is a U-shaped bend, and the length of the U-shaped bend on the ribbon-shaped strip is set to be a non-integer multiple of 1/4 of a vibration wavelength of the mesh nebulization sheet 1; and the bent structure of the strip-shaped ribbon between the FPC welding point 1.7 and the edge of the FPC 1.3 is a U-shaped bend, and the length of the U-shaped bend on the strip-shaped ribbon is set to be a non-integer multiple of 1/4 of the vibration wavelength of the mesh nebulization sheet 1. Since the FPC 1.3 has the characteristic of good bending property, the U-shaped bend thereof cannot be prefabricated. Therefore, as shown in Fig. 4 and Fig. 9, the location on an inner edge of a lower shell 3 that corresponds to a PIN mounting hole 3.3 is provided with a molded plate that has a width equal to or slightly greater than that of the strip-shaped ribbon extending out from the FPC 1.3, has a U-shaped cross section and has a length non-integer multiple of 1/4 of the vibration wavelength of the mesh nebulization sheet 1. Thus, after the mesh nebulization sheet 1 is installed in place in the housing, under the extrusion of an upper shell 2 and the molded plate in the lower shell 3, the strip-shaped ribbon extending out from the FPC 1.3 forms, between the FPC welding point 1.7 and the edge of the FPC 1.3, at least one U-shaped bend having a length non-integer multiple of 1/4 of the vibration wavelength of the mesh nebulization sheet 1.

Further, in some embodiments, the steel sheet 1.2 is circular, the FPC 1.3 and the piezoelectric ceramic 1.4 are both circular ring-shaped, the size of the FPC 1.3 is greater than the size of the piezoelectric ceramic 1.4, the FPC 1.3 completely covers the piezoelectric ceramic 1.4, and both sides of the FPC 1.3 are adhered to the steel sheet 1.2, so that the piezoelectric ceramic 1.4 is completely enclosed between the FPC 1.3 and the steel sheet 1.2.

In terms of process implementation, an appropriate amount of glue 1.1 may be applied in an assembly process of the mesh nebulization sheet 1, so that the glue 1.1 completely seals a side surface edge of the piezoelectric ceramic 1.4 while adhering the steel sheet 1.2, the FPC 1.3 and the piezoelectric ceramic 1.4, thereby realizing complete encapsulation of the piezoelectric ceramic 1.4 by the steel sheet 1.2 and the FPC 1.3, thus avoiding the possibility of precipitation of substances contained in the piezoelectric ceramic 4 during the use of the mesh nebulization sheet 1, improving the safety of the mesh nebulization sheet 1, and also reducing restrictions on the model selection of the piezoelectric ceramic material in the production of the mesh nebulization sheet 1.

Specifically, in the embodiment, the housing is circular ring-shaped and includes a circular ring-shaped upper shell 2 and a circular ring-shaped lower shell 3, the mesh nebulization sheet 1 is assembled at the annular center of the housing, at least three nebulization sheet positioning ribs I 2.1 protruding from an inner edge of the housing are uniformly disposed on an annular inner edge of the upper shell 2, the nebulization sheet positioning ribs I have consistent outlines and are all circular arc-shaped, the lower shell 2 is provided with at least three nebulization sheet positioning ribs II 3.1 having completely consistent outlines and opposite clamping directions at locations corresponding to the nebulization sheet positioning ribs I 2.1 of the upper shell 3, an annular outer edge of the lower shell 3 is a certain thickness higher than an annular inner edge thereof, and after the lower shell 3 is assembled with the upper shell 2, the lower shell 3 completely wraps the outer edge of the upper shell 2. In some embodiments, the height of the annular outer edge on the lower shell 3 higher than the annular inner edge thereof is exactly the thickness of the upper shell 2.

The specific structures of the nebulization sheet positioning rib I 1.1 and the nebulization sheet positioning rib II 3.1 are as follows: the nebulization sheet positioning rib I 1.1 and the nebulization sheet positioning rib II 3.1 are disposed at corresponding locations, and have completely consistent outlines, but opposite clamping directions. The surfaces of the nebulization sheet positioning rib I 1.1 and the nebulization sheet positioning rib II 3.1 facing the mesh nebulization sheet 1 are provided with flat convex surfaces, that is, nebulization sheet clamping surfaces, which function to touch the surface of the mesh nebulization sheet 1 and clamp the mesh nebulization sheet 1 together with a corresponding nebulization sheet clamping surface. Another convex surface facing the circle center of the housing is disposed between the tail of the nebulization sheet clamping surface and the housing, that is, a limiting surface of the mesh nebulization sheet 1, and the limiting surface always maintains a set gap between the edge of the mesh nebulization sheet 1 and the inner edge of the housing. A clamping opening exactly equal to the thickness of the mesh nebulization sheet 1 is present between the nebulization sheet clamping surface of the nebulization sheet positioning rib I 2.1 and the nebulization sheet clamping surface of the nebulization sheet positioning rib II 3.1, therefore the nebulization sheet positioning rib I 2.1 and the nebulization sheet positioning rib II 3.1 can exactly clamp the edge of the mesh nebulization sheet 1, and meanwhile, clamping points maintain a pressure close to 0; and moreover, under the action of the limiting surface, an appropriate gap is maintained between the edge of the mesh nebulization sheet 1 and the inner edge of the housing, thereby maximally reducing the restrictions of the housing on the working of the mesh nebulization sheet 1, and avoiding the problems of reduced nebulization ability and energy loss caused by the encapsulation of the mesh nebulization sheet.

The edge of the upper shell 2 is provided with at least one upper shell positioning groove 2.2, the inner side of the annular outer edge of the lower shell 3 is correspondingly provided with a lower shell positioning protrusion 3.2, and the shape and size of the lower shell positioning protrusion 3.2 completely match those of the upper shell positioning groove 2.2. The upper shell positioning groove 2.2 and the lower shell positioning protrusion 3.2 are jointly used for indicating a relative location between the upper shell 2 and the lower shell 3 during installation when the housing is assembled, thereby simplifying the assembly of the housing and preventing assembly errors.

The annular inner edge of the lower shell 3 is also provided with a certain height, so that an annular space between the annular outer edge of the lower shell 3 and the annular inner edge thereof forms a glue dispensing groove 3.4, as shown in Fig. 9, for injecting glue to bond various portions of the mesh nebulization unit assembly into a whole. In order to enhance the strength of the housing to better protect the mesh nebulization sheet 1, reinforcing ribs may further be disposed in the glue dispensing groove 3.4. Considering the perspectives of manufacturing process and production cost, the housing is generally made by using an injection molding process, and preferred materials include polypropylene PP, polyphenylsulphone PPSU, polyethyleneimine PEI, etc; and of course, other hard materials meeting medical use requirements may also be used.

In some embodiments, an inner bottom surface of the glue dispensing groove 3.4 is provided with a PIN mounting hole 3.3, the PIN mounting hole 3.3 is a hollow cylinder extending downwards starting from the bottom surface of the glue dispensing groove 3.4, a hollow inner diameter of the hollow cylinder matches an outer diameter of the inserted PIN 4, and the PIN 4 is provided with a pin cap having a diameter greater than a diameter of a pin foot, thereby ensuring that the PIN 4 does not fall off after being inserted into the PIN mounting hole 3.3. In some embodiments, the inner diameter of the top of the hollow cylinder is greater, and the inner diameter of the lower part of the hollow cylinder is smaller for matching and placing the PIN 4. The PIN 4 is used for connecting an external power supply, the steel sheet welding point 1.6 and the FPC welding point 1.7, so as to supply power to the mesh nebulization sheet 1.

When the mesh nebulization unit assembly is specifically assembled, first, the PIN 4 is inserted into the PIN mounting hole 3.3 on the lower shell 3. Then, glue 1.1 is injected into the glue dispensing groove 3.4 of the lower shell 3, the mesh nebulization sheet 1 is then laid flat at the annular inner center of the lower shell 3, so that the edge of the mesh nebulization sheet 1 is in contact with the nebulization sheet positioning ribs II 3.1, and the steel sheet welding point 1.6 and the FPC welding point 1.7 are connected with the PIN 4. Finally, according to the indication of the upper shell positioning groove 2.2 and the lower shell positioning protrusion 3.2, the upper shell 2 is covered on the lower shell 3 to complete the assembly of the entire mesh nebulization unit assembly. In a nebulization work, after the PIN 4 is connected to the power supply, the piezoelectric ceramic 1.4 generates mechanical energy under an excitation of an electrical signal source, so as to drive the steel sheet 1.2 to vibrate. The nebulization sheet positioning ribs I 2.1 and the nebulization sheet positioning ribs II 3.1 in the housing are only clamped at the edge of the mesh nebulization sheet, a pressure close to 0 is maintained for clamping points, and the reset of the mesh nebulization sheet 1 is not in contact with the housing, thereby maximally reducing the influence of the housing on the working of the mesh nebulization sheet 1, and avoiding the problems of reduced nebulization ability and energy loss caused by the encapsulation of the mesh nebulization sheet 1.

In addition to the wired power supply mode of connecting the PIN 4, the mesh nebulization unit assembly in the present disclosure further has an alternative solution of wireless power supply, and the specific structure is as follows:
the mesh nebulization unit assembly is provided with an electric energy wireless receiving apparatus, the electric energy wireless receiving apparatus is completely encapsulated in the housing, the electric energy wireless receiving apparatus is connected with the mesh nebulization sheet 1, and is used for receiving electric energy emitted by an external electric energy wireless sending apparatus, so as to realize wireless power supply to the mesh nebulization sheet 1. The wireless power supply mesh nebulization unit assembly may further improve the waterproof and dustproof performance of the mesh nebulization unit assembly, and may further effectively prolong the service life of the mesh nebulization unit assembly.

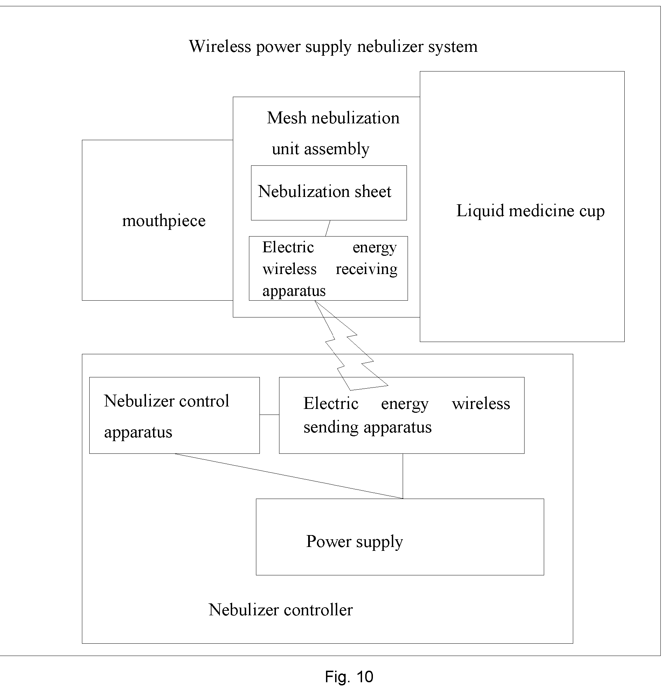

Referring to Fig. 10, an embodiment of a wireless power supply nebulizer system in the present disclosure includes a nebulization unit assembly, a liquid medicine cup, a mist nozzle and a nebulizer controller, wherein the nebulization unit assembly is the above mesh nebulization unit assembly provided with the electric energy wireless receiving apparatus, the nebulizer controller is provided with an electric energy wireless sending apparatus therein, the electric energy wireless sending apparatus is used for providing electric energy for the electric energy wireless receiving apparatus in the mesh nebulization unit assembly by means of the control of the nebulizer controller, and the electric energy wireless receiving apparatus drives the mesh nebulization sheet 1 in the mesh nebulization unit assembly to work. Specifically, the nebulizer controller further includes a nebulizer control apparatus and a power supply.

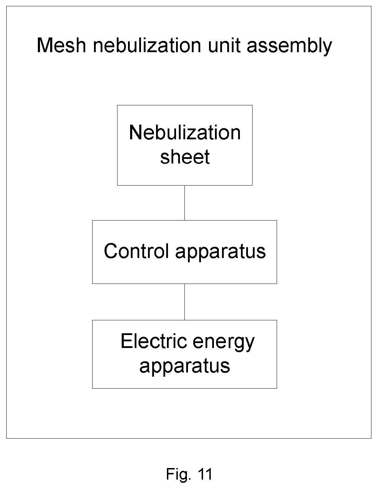

Referring to Fig. 11, an embodiment of an intelligent control mesh nebulization unit assembly in the present disclosure mainly increases a control apparatus and an electric energy apparatus on the basis of the above mesh nebulization unit assembly.

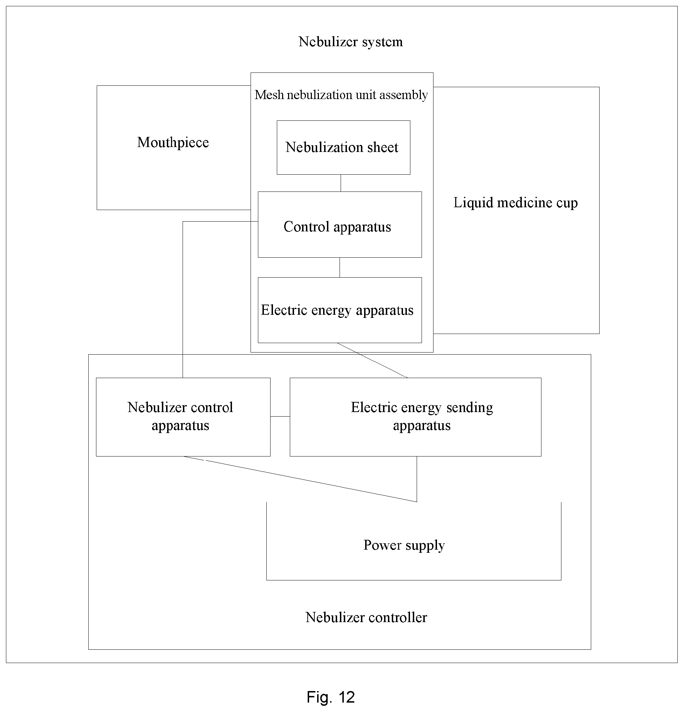

Referring to Fig. 12, an embodiment of an intelligent control nebulizer system in the present disclosure includes a nebulization unit assembly, a liquid medicine cup, a mist nozzle and a nebulizer controller, wherein the nebulization unit assembly is the above intelligent control mesh nebulization unit assembly, the intelligent control mesh nebulization unit assembly is additionally provided with a control apparatus and an electric energy apparatus, the control apparatus includes a communication module, a single chip microcomputer and a control module running thereon, the control apparatus is used for performing wired or wireless communication with the nebulizer controller, so as to control the mesh nebulization sheet 1 in the mesh nebulization unit assembly to work; and the electric energy apparatus uses a PIN wired connection power supply mode, a wireless power supply mode of the electric energy wireless receiving apparatus, or a power supply mode of a rechargeable battery to realize partial or complete power supply to the control apparatus and the mesh nebulization sheet 1. In some embodiments, a circuit switch controlled by the control apparatus is disposed between the mesh nebulization sheet 1 and the electric energy apparatus, and the control apparatus controls the circuit switch to turn on a driving circuit of the mesh nebulization sheet 1 and start the mesh nebulization sheet 1. Specifically, the nebulizer controller includes an electric energy sending apparatus, a nebulizer control apparatus and a power supply.

Further improvements: the communication module in the control apparatus is a wireless communication module, the nebulizer controller is provided with a wireless communication module and an electric energy wireless sending apparatus therein, the electric energy wireless receiving apparatus and the control apparatus are completely encapsulated in the housing in the mesh nebulization unit assembly, the electric energy wireless sending apparatus is used for providing electric energy for the electric energy wireless receiving apparatus in the mesh nebulization unit assembly by means of the control of the nebulizer controller, and the electric energy wireless receiving apparatus drives the mesh nebulization sheet in the mesh nebulization unit assembly to work.

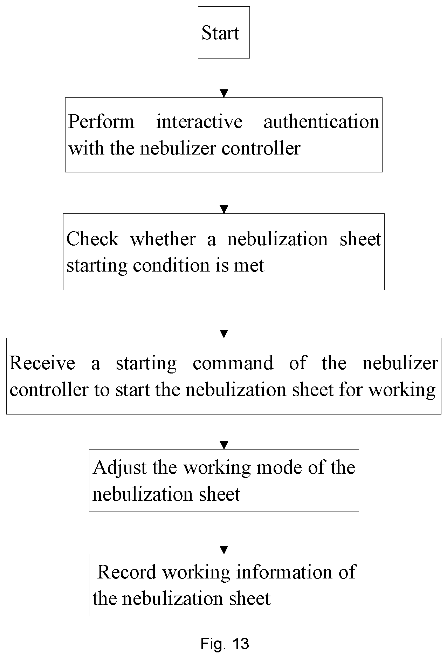

Referring to Fig. 13 to Fig. 15, an embodiment of a control method of the intelligent control nebulizer system in the present disclosure includes the following steps:
step 1: after the nebulizer system is powered on and started, the mesh nebulization unit assembly exchanging identity authentication information with the nebulizer controller by means of the communication module, so as to confirm whether the other party meets identity requirements for a connection work (e.g., a manufacturer of the mesh nebulization unit assembly may build in a list of allowed-to-connect nebulizer controllers in a program), and only when the nebulizer controller meets a preset allowed-to-use identity condition (e.g., only when the nebulizer controller is in an allowed list), the mesh nebulization unit assembly may complete an identity authentication operation and enter the next step; and otherwise, sending a refuse-to-work warning to the nebulizer controller, and prompting a cause in the warning message;
step 2: checking the current state of the mesh nebulization sheet 1, and according to a preset starting condition of the mesh nebulization sheet 1, determining whether the mesh nebulization sheet 1 is in an allowed-to-start state, wherein the starting condition of the mesh nebulization sheet 1 includes whether the service life (e.g., the number of times of use, or the duration of use or the duration of warranty) of the mesh nebulization sheet 1 has expired, and whether there is an abnormal record in an operation log that affects the working of the mesh nebulization sheet 1; when the check is passed and the starting condition of the mesh nebulization sheet is met, notifying the nebulizer controller that the mesh nebulization unit assembly is ready, and entering the next step to wait for a control command from the nebulizer controller; and otherwise, sending a refuse-to-work warning to the nebulizer controller, and prompting a cause in the warning message;
step 3: waiting for the control command of the nebulizer controller, and after receiving a command from the nebulizer controller to control the mesh nebulization sheet to start, starting the mesh nebulization sheet 1 to start working;
step 4: during the working of the mesh nebulization sheet 1, the control apparatus checking, in real time, whether working parameters of a driving circuit of the mesh nebulization sheet 1 are optimal, wherein the working parameters include driving power and working frequency; and adjusting input parameters of the driving circuit of the mesh nebulization sheet 1 according to a preset condition, so as to ensure that the mesh nebulization sheet 1 works in an optimal state; and
step 5: the control apparatus monitoring a working state of the mesh nebulization sheet 1, and recording working conditions and log information of the mesh nebulization sheet 1 in real time, including the number of times of use, time information of each use, operation information of the mesh nebulization sheet, and the information of a corresponding nebulizer controller (including a controller model, an identity identifier, a manufacturer, or the like).

In step 3, one implementation of starting the mesh nebulization sheet 1 to work is as follows: a circuit switch controlled by the control apparatus is disposed between the mesh nebulization sheet 1 and the electric energy apparatus, and the control apparatus controls the circuit switch to turn on the driving circuit of the mesh nebulization sheet 1 and start the mesh nebulization sheet 1; and
In step 4, one solution is: after the circuit switch is disposed in step 3, a circuit voltage transformation and frequency conversion apparatus is added, and the control apparatus controls the circuit voltage transformation and frequency conversion apparatus to control input current.

In order to better implement the above control method, the following detailed explanations and descriptions are given:
Information used during the interactive authentication between a storage assembly of the control apparatus and the nebulizer controller includes, but is not limited to, product identification number information of the unit assembly (the information is a unique code of the unit assembly, and is used for identifying each unit assembly), identity identification information (a segment of encrypted information that is built in by a manufacturer, matches a product identification number, and is used for verifying the product identification number information of the unit assembly), manufacturer information, model information, working parameter information of the nebulization sheet (i.e., working parameters, such as driving power and working frequency, required for the working of a nebulization sheet built in a controller driving assembly), a production date of the unit assembly, and use restriction information of the unit assembly, and the like, which are used for the check in the foregoing step 1 and step 2. The use restriction information defines use conditions of the unit assembly, for example, the service life (e.g., the number of times of use, or the duration of use, or the duration of the warranty), the list of allowed-to-connect nebulizer controllers, verification method information, conditions such as ambient temperature and humidity required when the unit assembly is used, or medicine information which the assembly may match, etc.

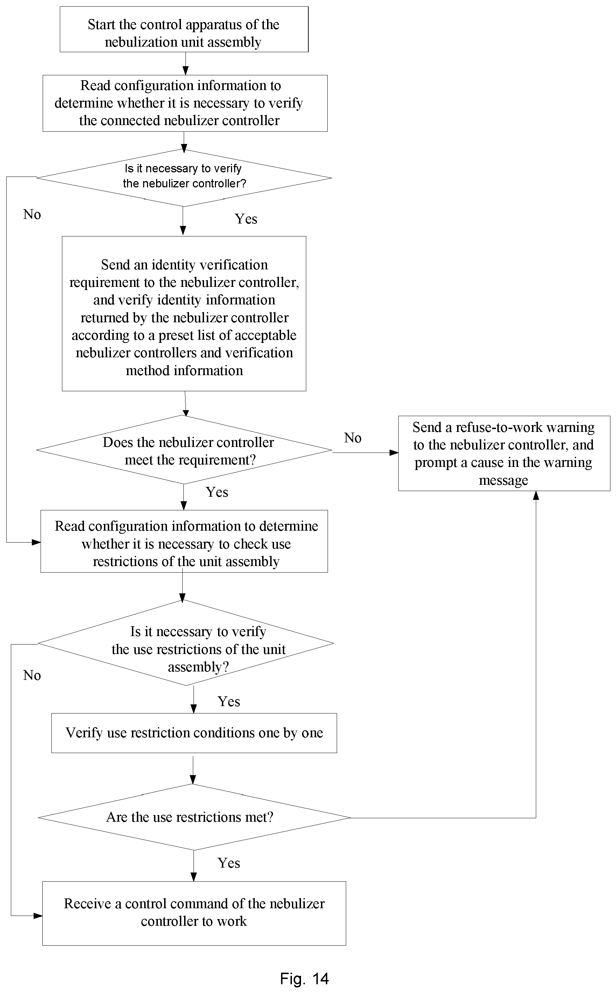

One of the key technical aspects of the present disclosure is to prevent the abuse of the nebulization sheet. Fig. 14 provides a detailed control flow of the intelligent control mesh nebulization unit assembly for completing the step 1 and the step 2 in Fig. 13. The intelligent control mesh nebulization unit assembly ensures that only the allowed nebulizer controller operates the nebulization unit assembly by checking whether the identity information of the connected nebulizer controller is in the list of allowed-to-connect nebulizer controllers. Furthermore, use restriction conditions are verified one by one to ensure that a nebulization work may be performed only when the nebulization unit assembly meets the use restriction conditions. The restriction conditions include own conditions of the intelligent mesh nebulization unit assembly, for example, the number of times that the intelligent mesh nebulization unit assembly has been used, the length of the used time, or any state that the intelligent mesh nebulization unit assembly itself may detect, for example, whether the mesh nebulization sheet 1 is blocked, whether dry braising happens, etc. The restriction conditions may also be conditions that the intelligent mesh nebulization unit assembly itself cannot detect, for example, nebulization medicine information and environment temperature and humidity, etc. In this case, the intelligent mesh nebulization unit assembly will require the connected nebulizer controller to provide relevant information. When the connected nebulizer controller cannot provide the relevant information, the verification of the use restriction conditions fails, a refuse-to-work warning is sent to the nebulizer controller, and a cause is prompted in the warning message.

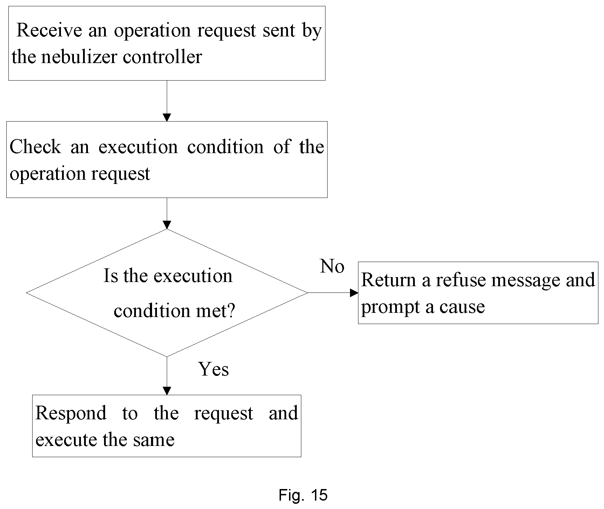

Correspondingly, the intelligent control mesh nebulization unit assembly also receives operation requests sent by the nebulizer controller connected thereto, and executes related operations. As shown in Fig. 15, for each received operation request, the intelligent control mesh nebulization unit assembly judges whether the operation request should be executed according to a preset condition, and executes the operation request sent by the nebulizer controller only when the preset condition is met. For example, identity request information sent by the nebulizer controller may be executed unconditionally, therefore when the nebulizer controller requires identity verification information from the intelligent control mesh nebulization unit assembly, the intelligent control mesh nebulization unit assembly returns, to the nebulizer controller, information such as product identification number information and identity identification information of the unit assembly. As another example, a starting operation request from the mesh nebulization sheet 1 sent by the nebulizer controller is conditional, and only when the nebulizer controller passes the verification and the use restriction conditions are met, the intelligent control mesh nebulization unit assembly may accept and execute the operation request.

Of course, the above description is not intended to limit the present disclosure, and the present disclosure is not limited to the above examples, and changes, modifications, additions or substitutions, made by those of ordinary skill in the art within the essential scope of the present disclosure, also fall within the protection scope of the present disclosure.

## Claims

1. A mesh nebulization unit assembly, comprising a mesh nebulization sheet, and a mesh hole being formed in a middle of the mesh nebulization sheet, wherein the mesh nebulization unit assembly further comprises a housing with an open pore formed in a middle, a periphery of the mesh nebulization sheet is encapsulated in the housing, the mesh hole in the middle of the mesh nebulization sheet is exposed from the open pore in the middle of the housing, at least three groups of nebulization sheet positioning ribs are disposed inside the housing to clamp an edge of the mesh nebulization sheet, a certain gap is maintained between a non-clamped portion of the mesh nebulization sheet and the housing, and a size of a clamping opening formed by each group of nebulization sheet positioning ribs is equal to or less than a thickness of the mesh nebulization sheet.

2. The mesh nebulization unit assembly according to claim 1, wherein the housing is made of a hard material, a nebulization sheet clamping surface of the each group of nebulization sheet positioning ribs is made of elastic soft rubber, and the size of the clamping opening formed by the each group of nebulization sheet positioning ribs is slightly less than the thickness of the mesh nebulization sheet.

3. The mesh nebulization unit assembly according to claim 1 or 2, wherein the mesh nebulization sheet is composed of a steel sheet provided with the mesh hole at a center, an FPC and a piezoelectric ceramic, a ribbon-shaped strip of an integrated structure extends out from an edge of the steel sheet, a steel sheet welding point for connecting with a PIN is disposed at a tail end of the ribbon-shaped strip, and the ribbon-shaped strip is provided with a bent structure between the steel sheet welding point and the edge of the steel sheet; and a strip-shaped ribbon of an integrated structure also extends out from an edge of the FPC, an FPC welding point for connecting with the PIN is disposed at a tail end of the strip-shaped ribbon, and the strip-shaped ribbon is provided with a bent structure between the FPC welding point and the edge of the FPC.

4. The mesh nebulization unit assembly according to claim 3, wherein the bent structure of the ribbon-shaped strip between the steel sheet welding point and the edge of the steel sheet is a U-shaped bend, and a length of the U-shaped bend on the ribbon-shaped strip is set to be a non-integer multiple of 1/4 of a vibration wavelength of the mesh nebulization sheet; and the bent structure of the strip-shaped ribbon between the FPC welding point and the edge of the FPC is a U-shaped bend, and a length of the U-shaped bend on the strip-shaped ribbon is set to be a non-integer multiple of 1/4 of the vibration wavelength of the mesh nebulization sheet.

5. The mesh nebulization unit assembly according to claim 3, wherein the steel sheet is circular, the FPC and the piezoelectric ceramic are both circular ring-shaped, a size of the FPC is greater than a size of the piezoelectric ceramic, the FPC completely covers the piezoelectric ceramic, and both sides of the FPC are adhered to the steel sheet, so that the piezoelectric ceramic is completely enclosed between the FPC and the steel sheet.

6. The mesh nebulization unit assembly according to claim 1 or 2, wherein the housing is circular ring-shaped and comprises a circular ring-shaped upper shell and a circular ring-shaped lower shell, the mesh nebulization sheet is assembled at an annular center of the housing, at least three nebulization sheet positioning ribs I protruding from an inner edge of the housing are uniformly disposed on an annular inner edge of the upper shell, the at least three nebulization sheet positioning ribs have consistent outlines and are all circular arc-shaped, the lower shell is provided with nebulization sheet positioning ribs II having completely consistent outlines and opposite clamping directions at locations corresponding to the nebulization sheet positioning ribs of the upper shell, an annular outer edge of the lower shell is a certain thickness higher than an annular inner edge thereof, and after the lower shell is assembled with the upper shell, the lower shell completely wraps the outer edge of the upper shell.

7. The mesh nebulization unit assembly according to claim 5, wherein the housing is circular ring-shaped and comprises a circular ring-shaped upper shell and a circular ring-shaped lower shell, the mesh nebulization sheet is assembled at an annular center of the housing, at least three nebulization sheet positioning ribs I protruding from an inner edge of the housing are uniformly disposed on an annular inner edge of the upper shell, the at least three nebulization sheet positioning ribs have consistent outlines and are all circular arc-shaped, the lower shell is provided with nebulization sheet positioning ribs II having completely consistent outlines and opposite clamping directions at locations corresponding to the nebulization sheet positioning ribs of the upper shell, an annular outer edge of the lower shell is a certain thickness higher than an annular inner edge thereof, and after the lower shell is assembled with the upper shell, the lower shell completely wraps the outer edge of the upper shell.

8. The mesh nebulization unit assembly according to claim 7, wherein an edge of the upper shell is provided with at least one upper shell positioning groove, an inner side of the annular outer edge of the lower shell is correspondingly provided with a lower shell positioning protrusion, and the shape and size of the lower shell positioning protrusion completely match those of the upper shell positioning groove.

9. The mesh nebulization unit assembly according to claim 7 or 8, wherein the annular inner edge of the lower shell is also provided with a certain height, so that an annular space between the annular outer edge of the lower shell and the annular inner edge thereof forms a glue dispensing groove for injecting glue to bond various portions of the mesh nebulization unit assembly into a whole.

10. The mesh nebulization unit assembly according to claim 9, wherein an inner bottom surface of the glue dispensing groove is provided with a PIN mounting hole, the PIN mounting hole is a hollow cylinder extending downwards starting from the inner bottom surface of the glue dispensing groove, a hollow inner diameter of the hollow cylinder matches an outer diameter of the inserted PIN, and the PIN is provided with a pin cap having a diameter greater than a diameter of a pin foot.

11. The mesh nebulization unit assembly according to claim 1 or 2, wherein the mesh nebulization unit assembly is provided with an electric energy wireless receiving apparatus, the electric energy wireless receiving apparatus is completely encapsulated in the housing, the electric energy wireless receiving apparatus is used for receiving electric energy emitted by an external electric energy wireless sending apparatus, so as to realize wireless power supply to the mesh nebulization sheet.

12. A wireless power supply nebulizer system, comprising a nebulization unit assembly, a liquid medicine cup, a mist nozzle and a nebulizer controller, wherein the nebulization unit assembly is the mesh nebulization unit assembly according to claim 11, the nebulizer controller is provided with an electric energy wireless sending apparatus, the electric energy wireless sending apparatus is used for providing electric energy for the electric energy wireless receiving apparatus in the mesh nebulization unit assembly by means of a control of the nebulizer controller, and the electric energy wireless receiving apparatus drives the mesh nebulization sheet in the mesh nebulization unit assembly to work.

13. An intelligent control nebulizer system, comprising a nebulization unit assembly, a liquid medicine cup, a mist nozzle and a nebulizer controller, wherein the nebulization unit assembly is the mesh nebulization unit assembly according to any one of claims 1-10, the mesh nebulization unit assembly further comprises a control apparatus and an electric energy apparatus, the control apparatus comprises a communication module, a single chip microcomputer and a control module running thereon, and the control apparatus is used for performing wired or wireless communication with the nebulizer controller, so as to control the mesh nebulization sheet in the mesh nebulization unit assembly to work; and the electric energy apparatus uses a PIN wired connection power supply mode, a wireless power supply mode of an electric energy wireless receiving apparatus, or a power supply mode of a rechargeable battery to realize partial or complete power supply to the control apparatus and the mesh nebulization sheet.

14. The intelligent control nebulizer system according to claim 13, wherein the communication module in the control apparatus is a wireless communication module, the nebulizer controller is provided with the wireless communication module and an electric energy wireless sending apparatus, the electric energy wireless receiving apparatus and the control apparatus are completely encapsulated in the housing in the mesh nebulization unit assembly, the electric energy wireless sending apparatus is used for providing electric energy for the electric energy wireless receiving apparatus in the mesh nebulization unit assembly by means of the control of the nebulizer controller, and the electric energy wireless receiving apparatus drives the mesh nebulization sheet in the mesh nebulization unit assembly to work.

15. A control method of the intelligent control nebulizer system according to claim 13, wherein the control method comprises following steps:
step 1: after the nebulizer system is powered on and started, the mesh nebulization unit assembly exchanging identity authentication information with the nebulizer controller by means of the communication module, so as to confirm whether the other party meets identity requirements of a connection work, and only when the nebulizer controller meets a preset allowed-to-use identity condition, the mesh nebulization unit assembly can complete an identity authentication operation and enter the next step; and otherwise, sending a refuse-to-work warning to the nebulizer controller, and prompting a cause in a warning message;
step 2: checking a current state of the mesh nebulization sheet, and according to a preset starting condition of the mesh nebulization sheet, determining whether the mesh nebulization sheet is in an allowed-to-start state, wherein the starting condition of the mesh nebulization sheet comprises whether a service life of the mesh nebulization sheet has expired, and whether there is an abnormal record in an operation log that affects a working of the mesh nebulization sheet; when a check is passed and the starting condition of the mesh nebulization sheet is met, notifying the nebulizer controller that the mesh nebulization unit assembly is ready, and entering a next step to wait for a control command from the nebulizer controller; and otherwise, sending a refuse-to-work warning to the nebulizer controller, and prompting a cause in the warning message;
step 3: waiting for the control command of the nebulizer controller, and after receiving a command from the nebulizer controller to control the mesh nebulization sheet to start, starting the mesh nebulization sheet to start working;
step 4: during a working of the mesh nebulization sheet, the control apparatus checking, in real time, whether working parameters of a driving circuit of the mesh nebulization sheet are optimal, wherein the working parameters comprise driving power and working frequency; and adjusting input parameters of the driving circuit of the mesh nebulization sheet according to a preset condition, so as to ensure that the mesh nebulization sheet works in an optimal state; and
step 5: the control apparatus monitoring a working state of the mesh nebulization sheet, and recording working conditions and log information of the mesh nebulization sheet in real time, comprising the number of times of use, time information of each use, operation information of the mesh nebulization sheet, and information of a corresponding nebulizer controller.
